(19) Europäisches Patentamt / European Patent Office / Office européen des brevets

(11) **EP 1 868 778 B1**

(12) **EUROPEAN PATENT SPECIFICATION**

(45) Date of publication and mention
of the grant of the patent:
**03.03.2010 Bulletin 2010/09**

(51) Int Cl.:
*B26B 21/44* (2006.01)   *A61K 8/46* (2006.01)
*A61Q 9/02* (2006.01)   *C11D 1/12* (2006.01)

(21) Application number: **06723919.4**

(22) Date of filing: **23.03.2006**

(86) International application number:
**PCT/EP2006/002959**

(87) International publication number:
**WO 2006/108522 (19.10.2006 Gazette 2006/42)**

(54) **RAZOR HEAD WITH MILD CLEANSING COMPOSITION AS A SHAVING AID**

RASIERKOPF MIT MILDER REINIGUNGSZUSAMMENSETZUNG WIE ZUM BEISPIEL
RASIERHILFE

TETE DE RASOIR CONTENANT UNE COMPOSITION NETTOYANTE DOUCE EN TANT
QU'AUXILIAIRE DE RASAGE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **12.04.2005 US 103769**

(43) Date of publication of application:
**26.12.2007 Bulletin 2007/52**

(73) Proprietors:
• **Unilever PLC
London
EC4Y 0DY (GB)**
Designated Contracting States:
**CY GB IE**
• **Unilever N.V.
3013 AL Rotterdam (NL)**
Designated Contracting States:
**AT BE BG CH CZ DE DK EE ES FI FR GR HU IS IT
LI LT LU LV MC NL PL PT RO SE SI SK TR**

(72) Inventors:
• **SLAVTCHEFF, Craig Stephen,
Unilever Japan K.K
Haga-gun, Tochigi 321-3325 (JP)**
• **FOMENKO, Sandra Dawn
Milford, Connecticut 06460 (US)**
• **ABBAS, Syed H.,
Unilever Home & Personal Care USA
Trumbull, Connecticut 06611 (US)**

(74) Representative: **James, Helen Sarah et al
Unilever Patent Group
Colworth House
Sharnbrook
Bedford
MK44 1LQ (GB)**

(56) References cited:
| WO-A-01/65964 | WO-A-2004/089320 |
| US-A- 3 823 185 | US-A- 4 231 904 |
| US-A- 4 562 644 | US-A- 4 695 395 |
| US-A- 5 349 750 | US-A- 5 653 970 |
| US-A1- 2002 137 643 | US-A1- 2003 083 210 |
| US-A1- 2003 208 915 | US-A1- 2005 011 073 |
| US-A1- 2005 028 370 | US-B1- 6 298 558 |
| US-B1- 6 458 751 | US-B1- 6 849 585 |

## Description

The present invention relates to a razor head suitable for personal shaving. In particular, it relates to a razor head associated with a cleansing composition that is mild to the skin and is easily manufactured.

A personal or safety razor typically includes a disposable razor cartridge mounted in a reusable handle, or a handle and a cartridge combined into a unitary disposable unit. Most razor cartridges include a frame, at least one razor blade, and a quantity of shaving aid material attached to or held within the frame to enhance the shaving process. The shaving aid material facilitates the movement of the razor blade over the skin and/or aids in the removal in advance of the blade assembly or simultaneously with the blade assembly of hair from the skin. Prior art shaving aid materials include lubricating agents, drag-reducing agents, depilatory agents, cleansing agents, medicinal agents, and the like. Prior art shaving aids that are also cleansing agents typically have consisted of soap and/or synthetic detergents that are either harsh to the skin, are disadvantageous to manufacture or both.

U.S. Patent No. 4,944,090 issued to Sumnall on July 31, 1990 discloses a razor head with a shaving aid sufficient to last the life of the razor without the need for supplementary shave creams or lubricants. U.S. Patent No. 4,074,429 issued to Roberts on Feb. 21, 1978 discloses a razor assembly with a soap cake and optional water reservoir for dispensing a lubricating lather in advance of the blade. U.S. Patent No. 6,584,690 issued to Orloff et al. on July 1, 2003 discloses a wet shaving assembly for simultaneously applying a shaving preparation and removing hair in a single step. The shaving cake used is soap and may contain optional lubricating or beneficial agents and fragrances. U.S. Patent Publication No. 2005/0011073 to Sandor et al. and published on Jan. 20, 2005 discloses a wet shaving assembly for simultaneously applying a shaving preparation and removing hair in a single step wherein the shaving cake may contain soap and/or synthetic detergents such as' alkyl glyceryl sulfonate and laureth-16.

U.S. Patent No. 4,170,821 issued to Booth on Oct. 16, 1979 discloses a water soluble shaving aid incorporated in a disposable razor blade cartridge which gradually dissolves during the act of wet shaving. The shaving aid may contain a cleaning agent such as a silicon polyethylene oxide block copolymer or sodium lauryl sulfate. U.S. Patent Publication No. 2004/0181943 to Kwiecken and published on Sep. 23, 2004 discloses a shaving aid composite including exfoliating elements or material.

The solid cleansing phase of the inventive razor head under actual use conditions is expected to be more easily manufactured due its higher Krafft point properties in a preferred embodiment. Furthermore it would be expected to show improvements in skin irritation, skin feel and similar consumer perceived benefits such as mildness, moisturization efficiency, deposition efficiency, cleansing efficiency, and other art recognized skin benefits based on changes from the baseline for these measurements, compared to prior art razor heads as quantified using the test methods described below and other art recognized test methods.

In one aspect of the invention, there is provided a razor head for personal shaving, including but not limited to the following: at least one blade member; a first skin engaging portion effectively positioned adjacent to said at least one blade for treating the skin of a user in advance of or simultaneously with the blade member contacting the skin of a user, the portion including and/or fluidly communicating with a mild cleansing base; and wherein the cleansing base includes at least one surfactant selected from C8 - C18 acyl isethionate(s).

In another aspect of the invention, there is provided a method for simultaneously shaving and moisturizing the skin with a razor including but not limited to the steps of:

1. providing a razor head including a cleansing base comprising about 1 % to 60 % by wt. of one or more surfactants selected from C8 - C18 acyl isethionate(s) and about 1 % to 60 % by wt. of a skin conditioning agent;

2. adding sufficient water to wet the cleansing base and the skin;

3. applying the razor head to the skin; and

4. moving the razor head across the skin to remove hair and coat the underlying skin with at least one skin conditioning agent.

Preferred embodiments of the invention will now be described by way of example with reference to the accompanying drawings wherein like figures represent like features, in which:

-    Fig. 1 is a top planar view showing one preferred embodiment of the razor head of the present invention;

-    Fig. 2 is a top planar view showing a second preferred embodiment of the razor head of the present invention;

-    Fig. 3 is a top planar view showing a third embodiment of the razor head of the present invention; and

- Fig. 4 is a top planar view showing a fourth preferred embodiment of the razor head of the present invention.

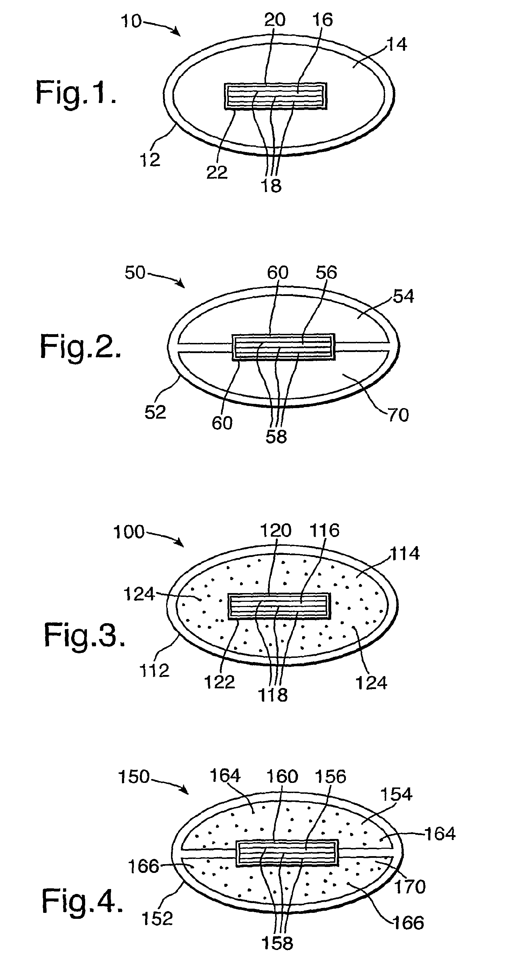

One preferred embodiment of the inventive razor head is depicted in Fig. 1. The inventive razor head 10 includes frame 12 which contains a first skin contacting portion including a cleansing phase 14 which may be a liquid or solid phase or a carrier phase saturated with a flowable cleansing phase. Also depicted in Fig. 1 is blade assembly 16 including razor blades 18 positioned in bore 20 adjacent to first skin contacting portion 14 and to wall 22.

Suitable carrier phase material includes woven and/or nonwoven fibrous medium, sponge medium, composite medium, or like medium which releasably contains a flowable cleansing phase whereby the carrier phase is either partially or fully saturated with a flowable liquid cleansing composition. In the case of a solid phase, the first skin contacting portion 14 and/or blade assembly 16 are movably situated with respect to each other within frame 12 or within separate frames situated in close proximity to each other. This is so that as the solid cleansing phase 14 wears away with use, the cleansing phase 14, blade assembly 16 or both can be automatically or manually repositioned to maintain skin contact for some period in advance of or during the shaving process.

Any mechanism or method suitable for moving the cleansing phase or blade assembly or both relative to each other to accomplish skin contact of the cleansing phase in advance of the blade assembly or simultaneously with the blade assembly may be used, such as a mechanical drive and/or gear mechanism, a ratcheting mechanism, a bias mechanism, a lever mechanism, a pneumatic mechanism, a rigid shaft mechanism, a frictional slide mechanism, or any combination or equivalent thereof. In use, the razor head may be exposed to water so as to activate the cleansing composition, or used with no added water.

A second preferred embodiment is depicted in Fig. 2. The inventive razor head 50 includes frame 52 which holds first skin contacting portion including a cleansing phase 54 alongside one side of blade assembly 56 and second skin contacting portion including after shave phase 70 positioned alongside the opposite side of blade assembly 56. Blade assembly 56 includes razor blades 58 positioned in bore 60 adjacent to first skin contacting portion 54 and second skin contacting portion 70. Frame 52 and bore 60 collectively separate first skin contacting portion 54 and second skin contacting portion 70. In other preferred embodiments, the skin contacting portions may be limited so as to only be coextensive with the length of the blade, and may be separated by a gap or abut each other if the first and second skin contacting portions are compatible with each other.

A third preferred embodiment of the inventive razor head is depicted in Fig. 3. The inventive razor head 100 includes frame 112 which contains a first skin contacting portion 114 having apertures 124 through which a liquid or flowable cleansing composition may be admitted onto the first skin contacting portion 114 so as to contact the skin of the user for some period while shaving. Also depicted in Fig. 3 is blade assembly 116 including razor blades 118 positioned in bore 120 adjacent to first skin contacting portion 114 and to wall 122.

In this embodiment, the flowable cleansing composition may be transported from a reservoir (not illustrated) within the razor head and/or handle or other storage container (not illustrated) via one or more conduits (not illustrated) communicating with apertures 124 by any suitable fluid transport mechanism such as capillary action, pressure differential, gravity or any combination or equivalent thereof, or by wicking material or other equivalent solid/liquid transport system for carrying the flowable cleansing composition to the skin while shaving in advance of the blade or simultaneously with the blade.

A fourth preferred embodiment is depicted in Fig. 4. The inventive razor head 150 includes frame 152 which holds first skin contacting portion 154 having apertures 164 through which a liquid or flowable cleansing composition may be admitted onto the first skin contacting portion 164 so as to contact the skin of the user for some period while shaving, analogous to the embodiment described in Fig. 3. First skin contacting portion 154 is situated alongside one side of blade assembly 156, and second skin contacting portion 170 is positioned alongside the opposite side of blade assembly 156. Second skin contacting portion 170 has apertures 166 through which a liquid or flowable after shave composition may be admitted onto the second skin contacting portion 170 so as to contact the skin of the user for some period after shaving analogous to the cleansing phase.

Blade assembly 156 includes razor blades 158 positioned in bore 160 adjacent to first skin contacting portion 154 and second skin contacting portion 170. Frame 152 and bore 160 collectively separate first skin contacting portion 154 and second skin contacting portion 170. In other preferred embodiments, the skin contacting portions may be limited so as to only be coextensive with the length of the blade, and may be separated by a gap or abut each other.

In operation, the user will slide the razor across the skin whereby the first skin contacting portion contacts the skin in advance of the blade assembly or simultaneously with the blade assembly. The optional after shave phase will contact the skin after the blade assembly contacts the skin, thus providing the after shave benefit to the skin just shaven. Analogous to the cleansing phase, the after shave phase may be a liquid or solid phase or a carrier phase saturated with a flowable phase and have the same transport characteristics discussed above for the cleansing phase.

In one aspect of the invention there is provided a razor head for personal shaving, including but not limited to the following: at least one blade member; a first skin engaging portion effectively positioned adjacent to said at least one blade for treating the skin of a user in advance of or simultaneously with the blade member contacting the skin of a user, the portion including and/or fluidly communicating with a mild cleansing base; and wherein the cleansing base includes at least one surfactant selected from C8 - C18 acyl isethionate(s), preferably having a Krafft point of about 20°C or greater, and preferably present in a total concentration of about 1 % to 60 % by wt. Preferably the cleansing phase has a zein value of less than 50. Preferably the blade member and cleansing base are situated within a single frame or in a plurality of frames positioned substantially adjacent to each other.

Advantageously the razor head further includes a second skin engaging portion effectively positioned adjacent to said at least one blade member for treating the skin of a user subsequent to and/or simultaneously with the blade member contacting the skin of a user, said portion including and/or fluidly communicating with an after shave base.

In a preferred embodiment, the inventive razor head has a cleansing phase that includes one or more hydrophillic and/or hydrophobic skin conditioning compounds in a total concentration range of about 1 % to 60 % by wt. In a preferred embodiment, the cleansing phase is in the form of a solid having a yield stress value from about 20 Kpa to 400 KPa at 25°C and 50 % RH.

Advantageously, the cleansing phase and/or the after shave phase or both contain an effective concentration of at least one compound selected from anti-acne actives, anti-wrinkle and anti-skin atrophy actives, skin barrier repair aids, cosmetic soothing aids, topical anesthetics, artificial tanning agents and accelerators, skin lightening actives, antimicrobial and antifungal actives, sunscreen actives, sebum stimulators, sebum inhibitors, antiperspirants, anti-glycation actives or mixtures thereof.

In a further preferred embodiment, the cleansing phase and/or after shave phase or both is/are in the form of a flowable liquid with a viscosity range of about 100 to 2000 cps at 25°C as measured using a rotary viscometer such as a Brookfield® or Haake® viscometer or the like. Preferably the flowable liquid includes potassium and/or sodium acyl isethionate, more preferably having about 1 to 10 moles of ethoxylation and most preferably being present at a concentration range of about 3 % to 15 % by weight.

Advantageously the skin conditioning compound is selected from polyols (such as polyethylene glycol), polyhydric alcohols, fatty acids, glyceride oil, mineral oil, petrolatum, glycerin, or blends thereof. Preferably the cleansing phase provides an aqueous slurry pH of about 4 to 8. More preferably the cleansing phase has less than about 10 % by wt. of soluble soap.

In another aspect of the invention, there is provided a method for simultaneously shaving and moisturizing the skin with a razor including but not limited to the steps of:

1. providing a razor head including a cleansing base comprising about 1 % to 60 % by wt. of one or more surfactants selected from C8 - C18 acyl isethionate(s) and about 1 % to 60 % by wt. of a skin conditioning agent;

2. adding sufficient water to wet the cleansing base and the skin;

3. applying the razor head to the skin; and

4. moving the razor head across the skin to remove hair and coat the underlying skin with at least one skin conditioning agent.

As discussed above, the inventive razor head under actual use conditions is expected to show improvements in skin softness, skin smoothness, and similar consumer perceived benefits such as after shave feel, mildness, moisturization efficiency, deposition efficiency, cleansing efficiency, etc. based on changes from the baseline for these measurements using razor heads with prior art shaving aid cleansing compositions, as quantified using the test methods described below. These skin benefit parameters can also be expressed quantitatively as the ratio of the inventive razor head response to the comparative razor head response. Where the magnitude of the inventive razor head benefit improvement is expected to exceed the numerical result of the comparative razor head, the observed ratio will be greater than 1.0; i.e. greater than 1.02, 1.05, 1.07, 1.1, 1.2, 1.3, 1.4, 1.5, 1.6, 1.7, 1.8, 1.9, or 2.0.

Table 1 below illustrates how various properties of a preferred embodiment of the inventive razor head are expected to compare to prior art razor heads with a cleansing phase that does not contain the inventive mild, isethionate surfactants. The test methods that may be used to measure the properties are provided below. In a preferred embodiment of the invention, the after shave phase will provide skin treatment benefits as part of the shaving process without the need for separate application of an after shave preparation to the skin.

Table 1a: Inventive razor head with solid cleansing phase A[1] vs. Comparative A1 [2] (solid phase soap razor head)

| Property | Ratio vs. Comparative A1 |
| --- | --- |
| Mildness | > 1 |

(continued)

| Property | Ratio vs. Comparative A1 |
|---|---|
| Moisturization | > 1 |
| Moisturizer deposition | > 1 |
| Softness | > 1 |
| Smoothness | > 1 |
| [1] Inventive A: see Table 1 [2] Comparative A1: Schick Intuition® razor with solid cleansing phase containing Sodium Palmitate, Sodium Cocoate, Sodium Isostearate, Water, Potassium Palmitate, Glycerin, Potassium Isostearate, Sodium Isostearoyl Lactylate, Sodium Coconut Alkyl Glyceryl Sulfonate Paste, Kaolin, Isostearic Acid, Sucrose Cocoate, Rosewood Oil, Almond Oil, Cedarwood Oil, Rose Oil, Titanium Dioxide, Sodium Chloride, Tocopheryl Acetate, Cocoa Butter, Tetrasodium Etidronate, Pentasodium Pentetate, Aloe Barbadensis | |

Table 2a: Inventive razor head with solid cleansing phase A vs. Comparative A2[3] (solid phase soap razor head with added DEA-Myristate and/or laureth 16 synthetic detergents)

| Property | Ratio vs. Comparative A2 |
|---|---|
| Mildness | $\geq 1$ |
| Moisturization | $\geq 1$ |
| Moisturizer deposition | $\geq 1$ |
| Softness | $\geq 1$ |
| Smoothness | $\geq 1$ |
| [3] Comparative A2: Sodium Palmitate, Sodium Cocoate, Sodium Isostearate, Water, Potassium Palmitate, Glycerin, Potassium Isostearate, Sodium Isostearoyl Lactylate, Sodium Coconut Alkyl Glyceryl Sulfonate Paste, Kaolin, Isostearic Acid, Sucrose Cocoate, Rosewood Oil, Almond Oil, Cedarwood Oil, Rose Oil, Titanium Dioxide, Sodium Chloride, Tocopheryl Acetate, Cocoa Butter, Tetrasodium Etidronate, Pentasodium Pentetate, Aloe Barbadensis with added DEA-Myristate and/or Alkyl Glyceryl Sulfonate and/or laureth 16 synthetic detergents. | |

Surfactant are an essential component of the cleansing phase of the inventive razor head. They are compounds that have hydrophobic and hydrophilic portions that act to reduce the surface tension of the aqueous solutions they are dissolved in. Useful surfactants can include soap(s), and non-soap anionic, nonionic, amphoteric, and cationic surfactant (s), and blends thereof in addition to the mild isethionate synthetic detergents of the present invention. The cleansing phase of the inventive razor head contains C8-C18 acyl isethionate surfactants having the general formula:

$$RC\text{-}O(O)\text{-}CH_2\text{-}CH_2\text{-}SO_3M^+$$

or

$$(RC\text{-}O(O)\text{-}CH_2\text{-}CH_2\text{-}SO_3)_2M^{++}$$

wherein R is an alkyl group having 8 to 18 carbons, and M is a mono or divalent cation such as, for example, sodium, potassium, ammonium, calcium and magnesium or other mono- and divalent cations. Preferably the isethionates have an average iodine value of less than 20.

Krafft point is an important consideration for selecting one or more primary surfactants for both the solid and liquid or flowable form of the cleansing phase of the preferred embodment of the invention. For the solid phase, it is essential to keep the Krafft point of this surfactant above 20, 21, 22 or 23°C to keep'the cleansing phase integrity, mush, wear, and processability under acceptable limits. Advantageously the maximum Krafft point is less than about 45°C. Typically the Krafft point of the isethionate surfactant will be under 23, 22, 21 or 20°C for the liquid cleansing phase of the invention.

Preferably acyl isethionates used in the solid cleansing phase embodment of the present invention are produced by a "DEFI" reaction where a mixture of a C8-C18, preferably C10 to C15 or C16-C18 fatty acids (e.g., lauric and coconut acid) reacts with alkali metal isethionate as follows:

$$RC-O(OH) + HO-CH_2-CH_2-SO_3^- M^+ \rightarrow RC-O(O)-CH_2-CH_2-SO_3M \text{ (plus residual starting materials)}$$

or

$$RC-O(OH) + (HO-CH_2-CH_2-SO_3)_2^- M^{++} \rightarrow (RC-O(O)-CH_2-CH_2-SO_3)_2M \text{ (plus residual starting materials)}.$$

The reaction is advantageously conducted at a stoichiometric ratio of about 1 to 1 to 2 to 1 fatty acid to isethionate using 0.01 % to 1 %, preferably 0.1 % to 0.4 % of total reactants by weight of a catalyst (e.g., zinc oxide, zirconium oxide, zinc isethionate or any Lewis acid including sulfuric acid, p-toluene sulfonic acid, sodium bisulfite etc.) at a temperature of about 150˚C to 250˚C, preferably about 200˚C to 250˚C for about 1 to 3 hours. It is often advantageous to use a relatively small amount of the final product (produced earlier) as an emulsifying agent for the reaction mixture to help speed up the reaction. The components of the reaction may be added in any order and, although yields may be better reacting one agent before another, any order of addition is contemplated.

One surfactant advantageously used in the solid cleansing phase of a preferred embodiment of this invention is sodium cocoyl isethionate with balanced chain length for desirable lathering and processing properties. For desirable lathering properties, 40 % to 95 % of the acyl isethionate is C8-C14. More preferably this range is from 50 % to 90 % or 60 % to 85 %, and most preferably it is from 70 % to 75 %. However too high a concentration C8-C14 acyl isethionates, which have a lower Krafft point than e.g. C16-C18 acyl isethionates, will result in a soft product that cannot be processed effectively by an extrusion process.

Further such bars cannot be stamped properly and will have many defects. Therefore a balance is necessary between lower Krafft point isethionates for lather, and higher Krafft point isethionates for efficient extrusion and stamping processability. $C_{16}$-$C_{18}$ acyl isethionates are advantageously in the range of 5 % to 40% or 35%, preferably in the range of 15 % to 30 %, and most preferably in the range of 23 % to 28 % for this embodiment. The most preferred ratio of C8-C14 isethionate to C16-C18 isethionate is about 3:1. As discussed above, surfactants with lower Krafft points can be used but at very low levels as co-surfactants in the solid cleansing composition.

For the liquid or flowable cleansing phase of another preferred embodiment of the invention, the preferred component is a potassium and/or sodium salt of C8-C18, preferably C8-C14 acyl isethionates with lower Krafft point than are optimally found in the solid cleansing phase and with preferably 1-10 moles of ethoxylation.

The cleansing phase of the inventive razor head may contain one or more non-soap anionic detergent(s) (syndets) other than acyl isethionates useful as co-surfactants. Preferably the syndet(s) have a zein value of 50 or less so as to provide mildness. Zein value may be measured using the test method described below. Advantageously such non-soap anionic detergent(s) or surfactant(s) may be used from about 1, 2, 3, 5, 10, 15, 20 or 30 % by wt. to about 40, 50 or 60 % by wt.

The anionic detergent active which may be used as a co-surfactant may be aliphatic sulfonate(s), such as a primary alkane (e.g., $C_8$-$C_{22}$) sulfonate(s), primary alkane (e.g., $C_8$-$C_{22}$) disulfonate(s), $C_8$-$C_{22}$ alkene sulfonate(s), $C_8$-$C_{22}$ hydroxyalkane sulfonate(s) or alkyl glyceryl ether sulfonate(s) (AGS); or aromatic sulfonate(s) such as alkyl benzene sulfonate.

The anionic may also be alkyl sulfate(s) (e.g., $C_{12}$-$C_{18}$ alkyl sulfate) or alkyl ether sulfate (including alkyl glyceryl ether sulfates). Among the alkyl ether sulfate(s) are those having the formula:

$$RO(CH_2CH_2O)_nSO_3M$$

wherein R is an alkyl or alkenyl having 8 to 18 carbons, preferably 12 to 18 carbons, n has an average value of greater than 1.0, preferably greater than 3, and M is a solubilizing cation such as sodium, potassium, ammonium or substituted ammonium. Ammonium and sodium lauryl ether sulfates are preferred.

The anionic may also be alkyl sulfosuccinate(s) (including mono- and dialkyl, e.g., $C_6$-$C_{22}$ sulfosuccinate(s)), alkyl and acyl taurate(s), alkyl and acyl sarcosinate(s), sulfoacetate(s), $C_8$-$C_{22}$ alkyl phosphate(s) and phosphate(s), alkyl phosphate ester(s) and alkoxyl alkyl phosphate ester(s), acyl lactate(s), $C_8$-$C_{22}$ monoalkyl succinate(s) and maleate(s), sulphoacetate(s), and alkyl glucoside(s) and the like.

Sulfosuccinates may be monoalkyl sulfosuccinates having the formula:

$$R^4O_2CCH_2CH(SO_3M)CO_2M$$

and amide-MEA sulfosuccinates of the formula;

$$R^4CONHCH_2CH_2O_2CCH_2CH(SO_3M)CO_2M$$

wherein $R^4$ ranges from $C_8$-$C_{22}$ alkyl and M is a solubilizing cation.

Sarcosinates are generally indicated by the formula:

$$R^1CON(CH_3)CH_2CO_2M$$

wherein $R^1$ ranges from $C_8$-$C_{20}$ alkyl and M is a solubilizing cation.

Taurates are generally identified by formula:

$$R^2CONR^3CH_2CH_2SO_3M$$

wherein $R^2$ ranges from $C_8$-$C_{20}$ alkyl, $R^3$ may be H or $C_1$-$C_4$ alkyl and M is a solubilizing cation.

The inventive razor head has a cleansing phase that may include low levels of fatty acid soap and preferably under 30, 20, 10, 8, 5, 3, 2, or 1 % by wt. of soap to improve mildness. The term "soap" is used here in its popular sense, i.e., the alkali metal or alkanol ammonium salts of aliphatic alkane or alkene monocarboxylic acids preferably having about 12 to 22 carbon atoms, more preferably about 12 to about 18 carbon atoms. They may be further described as alkali metal carboxylates of aliphatic hydrocarbons. Sodium, potassium, mono-, di- and tri-ethanol ammonium cations, or combinations thereof, are suitable for purposes of this invention.

In general, sodium soaps are used in the compositions of this invention, but from about 1 % to about 25 % of the soap may be potassium soaps. The soaps may contain unsaturation in accordance with commercially acceptable standards. Excessive unsaturation is normally avoided to minimize color and odor issues.

Soaps may be made by the classic kettle boiling process or modern continuous soap manufacturing processes, wherein natural fats and oils such as tallow or coconut oil or their equivalents are saponified with an alkali metal hydroxide using procedures well known to those skilled in the art. Alternatively, the soaps may be made by neutralizing fatty acids, such as lauric (C12), myristic (C14), palmitic (C16), or stearic (C18) acids with an alkali metal hydroxide or carbonate.

Fatty acids in this carbon number range may be added to the solid cleansing phase in a small quantity as a process aid to generate the desired level of liquid crystalline phase for acceptable processing.

One or more amphoteric surfactants may be used in this invention as a co-surfactant. Advantageously amphoteric surfactants may be used from about 1, 2 or 3 % by wt. to about 5, 6, 7, 10 or 12 % by wt. Such surfactants include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula:

$$R^1\text{-}[\text{-}C(O)\text{-}NH(CH_2)_n\text{-}]_m\text{-}N^+\text{-}(R^2)(R^3)X\text{-}Y$$

where $R^1$ is alkyl or alkenyl of 7 to 18 carbon atoms, $R^2$ and $R^3$ are each independently alkyl, hydroxyalkyl or carboxyalkyl of 1 to 3 carbon atoms, n is 2 to 4, m is 0 to 1, X is alkylene of 1 to 3 carbon atoms optionally substituted with hydroxyl, and Y is $-CO_2$- or $-SO_3$-.

Suitable amphoteric surfactants within the above general formula include simple betaines of formula:

$$R^1\text{-}N^+\text{-}(R^2)(R^3)CH_2CO_2^-$$

and amido betaines of formula:

$$R^1\text{-}CONH(CH_2)_n\text{-}N^+\text{-}(R^2)(R^3)CH_2CO_2^-$$

where n is 2 or 3.

In both formulae $R^1$, $R^2$ and $R^3$ are as defined previously. $R^1$ may in particular be a mixture of $C_{12}$ and $C_{14}$ alkyl groups derived from coconut oil so that at least half, preferably at least three quarters of the groups $R^1$ have 10 to 14 carbon atoms. $R^2$ and $R^3$ are preferably methyl.

A further possibility is that the amphoteric detergent is a sulphobetaine of formula:

$$R^1\text{-}N^+\text{-}(R^2)(R^3)(CH_2)_3SO_3^-$$

or

$$R^1\text{-}CONH(CH_2)_m\text{-}N^+\text{-}(R^2)(R^3)(CH_2)_3SO_3^-$$

where m is 2 or 3, or variants of these in which $-(CH_2)_3SO_3$ is replaced by $-CH_2C(OH)(H)CH_2SO_3^-$

In these formulae $R^1$, $R^2$ and $R^3$ are as discussed previously.

Amphoacetates and diamphoacetates are also intended to be covered in the zwitterionic and/or amphoteric compounds which are used such as e.g., sodium lauroamphoacetate, sodium cocoamphoacetate, and blends thereof, and the like.

One or more nonionic surfactants may also be used in the cleansing phase of the inventive razor head composition of the present invention. When present, nonionic surfactants may be used at levels as low as about 0.5, 1, 2 or 3 % by wt. and as high as about 5, 10, 15 or 20 % by wt.

The nonionics which may be used include in particularly the reaction products of compounds having a hydrophobic group and a reactive hydrogen atom, for example aliphatic alcohols, acids, amides or alkylphenols with alkylene oxides, especially ethylene oxide either alone or with propylene oxide.

Specific nonionic detergent compounds are alkyl ($C_6$-$C_{22}$) phenols ethylene oxide condensates, the condensation products of aliphatic ($C_8$-$C_{18}$) primary or secondary linear or branched alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxide, and the like.

The nonionic may also be a sugar amide, such as a polysaccharide amide. Specifically, the surfactant may be one of the lactobionamides described in U.S. Patent No. 5,389,279 to Au et al. titled "Compositions Comprising Nonionic Glycolipid Surfactants" issued February 14, 1995, which is hereby incorporated by reference, or it may be one of the sugar amides described in Patent No. 5,009,814 to Kelkenberg, titled "Use of N-Poly Hydroxyalkyl Fatty Acid Amides as Thickening Agents for Liquid Aqueous Surfactant Systems" issued April 23, 1991, hereby incorporated into the subject application by reference.

An optional component in compositions according to the invention is a cationic skin feel agent or polymer which may be a cationic skin conditioning agent, such as for example cationic celluloses or polyquarterium compounds.

Advantageously cationic skin feel agent(s) or polymer(s) are used from about 0.01, 0.1 or 0.2 % by wt. to about 1, 1.5 or 2.0 % by wt. Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200, and quaternary ammonium compounds such as alkyldimethylammonium halogenides.

A particularly suitable type of cationic polysaccharide polymer that can be used is a cationic guar gum derivative, such as guar hydroxypropyltrimonium chloride (commercially available from Rhone-Poulenc in their JAGUAR trademark series). Examples are JAGUAR C13S, which has a low degree of substitution of the cationic groups and high viscosity, JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency', medium viscosity guar having a low degree of substitution.

Particularly preferred cationic polymers are JAGUAR C13S, JAGUAR C15, JAGUAR C17 and JAGUAR C16 and JAGUAR C162, especially Jaguar C13S. Other cationic skin feel agents known in the art may be used provided that they are compatible with the inventive formulation.

Other preferred cationic compounds that are useful in the present invention include amido quaternary ammonium compounds such as quaternary ammonium propionate and lactate salts, and quaternary ammonium hydrolyzates of silk or wheat protein, and the like. Many of these compounds can be obtained as the Mackine™ Amido Functional Amines, Mackalene™ Amido functional Tertiary Amine Salts, and Mackpro® cationic protein hydrolysates from the McIntyre Group Ltd. (University Park, IL).

In a preferred embodiment of the invention having a hydrolyzed protein conditioning agent, the average molecular weight of the hydrolyzed protein is preferably about 2500. Preferably 90 % of the hydrolyzed protein is between a molecular weight of about 1500 to about 3500. In a preferred embodiment, MACKPRO™ WWP (i.e. wheat germ amido dimethylamine hydrolyzed wheat protein) is added at a concentration of 0.1 % (as is) in the bar. This results in a MACKPRO™ WWP "solids" of 0.035 % in the final bar formula for this embodiment.

One or more cationic surfactants may also be used in the cleansing phase of the inventive razor head. Advantageously cationic surfactants may be used from about 0.1, 0.5 or 1.0 % by wt. to about 1.5, 2.0 or 2.5 % by wt.

Examples of cationic detergents are the quaternary ammonium compounds such as alkyldimethylammonium halogenides.

Other suitable surfactants which may be used are described in U.S. Patent No. 3,723,325 to Parran Jr. titled "Detergent Compositions Containing Particle Deposition Enhancing Agents" issued March, 27, 1973, and "Surface Active Agents and Detergents" (Vol. I & II) by Schwartz, Perry & Berch, both of which are also incorporated into the subject application by reference.

In addition, the cleansing phase of the inventive razor head may include 0 to 15 % by wt. optional ingredients as follows: perfumes; sequestering agents, such as tetrasodium ethylenediaminetetraacetate (EDTA), EHDP or mixtures in an amount of 0.01 % to 1 %, preferably 0.01 % to 0.05 %; and coloring agents, opacifiers and pearlizers such as zinc stearate, magnesium stearate, $TiO_2$, EGMS (ethylene glycol monostearate) or Lytron 621 (Styrene/Acrylate copolymer) and the like; all of which are useful in enhancing the appearance or cosmetic properties of the product.

The compositions may further comprise preservatives such as dimethyloldimethylhydantoin (Glydant XL1000), parabens, sorbic acid etc., and the like. The compositions may also comprise coconut acyl mono- or diethanol amides as suds boosters, and strongly ionizing salts such as sodium chloride and sodium sulfate may also be used to advantage. Antioxidants such as, for example, butylated hydroxytoluene (BHT) and the like may be used advantageously in amounts of about 0.01 % or higher if appropriate.

Skin conditioning agents also termed emollients are advantageously used in the cleansing phase of the inventive razor head. Hydrophilic emollients including humectants such as polyhydric alcohols, e.g. glycerin and propylene glycol, and the like; polyols such as the polyethylene glycols listed below, and the like and hydrophilic plant extracts may be used. Advantageously humectants may be used from about 0.01, 0.2 or 1.0 % by wt. to about 3, 5 or 10 % by wt. in the solid phase cleansing composition and up to about 5, 10, 15, 20, 25, 30, 35, 40 % or more for the liquid cleansing phase cleansing composition. Humectants may also confer the ability for the solid cleansing phase to retain water.

| | |
|---|---|
| Polyox WSR-205 | PEG 14M, |
| Polyox WSR-N-60K | PEG 45M, or |
| Polyox WSR-N-750 | PEG 7M. |

Hydrophobic emollients may be used in the cleansing phase of the inventive razor head. Advantageously hydrophobic emollients may be used from about 5, 10 or 15 % by wt. to about 20, 25, 30, 35, 40, 45 % by wt. for the solid cleansing phase and from about 0.1, 0.5, or 1 % by wt. to about 3 5, 7, 9, 10, 15, 20, or 25 % by weight for the liquid cleansing phase.

The term "emollient" is defined as a substance which softens or improves the elasticity, appearance, and youthfulness of the skin (stratum corneum) by increasing its water content, and keeps it soft by retarding the decrease of its water content.

Useful hydrophobic emollients include the following:

(a) silicone oils and modifications thereof such as linear and cyclic polydimethylsiloxanes; amino, alkyl, alkylaryl, and aryl silicone oils;

(b) fats and oils including natural fats and oils such as jojoba, soybean, sunflower, rice bran, avocado, almond, olive, sesame, persic, castor, coconut, mink oils; cacao fat; beef tallow, lard; hardened oils obtained by hydrogenating the aforementioned oils; and synthetic mono, di and triglycerides such as myristic acid glyceride and 2-ethylhexanoic acid glyceride;

(c) waxes such as carnauba, spermaceti, beeswax, lanolin, and derivatives thereof;

(d) hydrophobic plant extracts;

(e) hydrocarbons such as liquid paraffin, petrolatum, microcrystalline wax, ceresin, squalene, pristan and mineral oil;

(f) higher fatty acids such as lauric, myristic, palmitic, stearic, behenic, oleic, linoleic, linolenic, lanolic, isostearic, arachidonic and poly unsaturated fatty acids (PUFA);

(g) higher alcohols such as lauryl, cetyl, stearyl, oleyl, behenyl, cholesterol and 2-hexydecanol alcohol;

(h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate;

(i) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, sweet almond, rose, cedarwood, limonene and terpenoid oils; and

(j) mixtures of any of the foregoing components, and the like.

Preferred hydrophobic emollient moisturizing agents are selected from fatty acids, di- and triglyceride oils, mineral oils, petrolatum, and mixtures thereof; with fatty acids being most preferred.

The Krafft point of a surfactant is defined as the temperature (or more precisely, the narrow temperature range) above which the solubility of a surfactant rises sharply. In other words, the Krafft point of a surfactant solution is the temperature below which the surfactant falls out of solution at concentrations above the critcal micelle concentration or CMC. Below the Krafft point the surfactant is incapable of solubilization and formulations become unstable. At this temperature the solubility of the surfactant becomes equal to the critical micelle concentration. It may be determined by locating the abrupt change in slope of a graph of the logarithm of the solubility against temperature or 1/T or can be rapidly estimated using the rapid estimation procedure described below.

The cleansing and/or after shave phase of the inventive razor head may contain particles that are greater than 50, 60, 70, 80, 90 or 100 microns in average diameter (or major axis length) that help remove dry skin. Not being bound by theory, the degree of exfoliation depends on the size and morphology of the particles. Large and rough particles are usually very harsh and irritating. Very small particles may not serve as effective exfoliants. Such exfoliants used in the art include natural minerals such as silica, talc, calcite, pumice, tricalcium phosphate; seeds such as rice, apricot seeds, etc; crushed shells such as almond and walnut shells; oatmeal; polymers such as polyethylene and polypropylene beads, flower petals and leaves; microcrystalline wax beads; jojoba ester beads, and the like. These exfoliants come in a variety of particle sizes and morphology ranging from micron sized to a few mm. They also have a range of hardness. Some examples are given in table A below.

Table A

| Material | Hardness (Mohs) |
| --- | --- |
| Talc | 1 |
| Calcite | 3 |
| Pumice | 4-6 |
| Walnut Shells | 3-4 |
| Dolomite | 4 |
| Polyethylene | ~1 |

Advantageously, optional active agents other than skin conditioning agents defined above may be added to the cleansing phase and/or after shave phase of the inventive razor head. These active ingredients may be advantageously selected from bactericides, vitamins, anti-acne actives; anti-wrinkle, anti-skin atrophy and skin repair actives; skin barrier repair actives; non-steroidal cosmetic soothing actives; artificial tanning agents and accelerators; skin lightening actives; sunscreen actives; sebum stimulators; sebum inhibitors; antiperspirants, anti-oxidants; protease inhibitors; skin tightening agents; anti-itch ingredients; hair growth inhibitors; 5-alpha reductase inhibitors; desquamating enzyme enhancers; anti-glycation agents; or mixtures thereof; and the like.

These active agents may be selected from water-soluble active agents, oil-soluble active agents, pharmaceutically-acceptable salts and mixtures thereof. The term "active agent" as used herein means personal care actives which can be used to deliver a benefit to the skin and/or hair and which generally are not used to confer a skin conditioning benefit, such are delivered by emollients as defined above.

The term "safe and effective amount", as used herein, means an amount of active agent high enough to modify the condition to be treated or to deliver the desired skin care benefit, but low enough to avoid serious side effects. The term "benefit," as used herein, means the therapeutic, prophylactic, and/or chronic benefits associated with treating a particular condition with one or more of the active agents described herein. What is a safe and effective amount of the active agent(s) will vary with the specific active agent, the ability of the active to penetrate through the skin, the age, health condition, and skin condition of the user, and other like factors.

Preferably the compositions of the present invention comprise from about 0.0001 % to 50 %, more preferably from about 0.05 % to 25 %, even more preferably about 0.1 % to 10 %, and most preferably about 0.1 % to 5 %, by weight of the active agent component(s).

A wide variety of active agent ingredients are useful herein and include those selected from anti-acne actives, anti-wrinkle and anti-skin atrophy actives, skin barrier repair aids, cosmetic soothing aids, topical anesthetics, artificial tanning agents and accelerators, skin lightening actives, antimicrobial and antifungal actives, sunscreen actives, sebum stimulators, sebum inhibitors, anti-glycation actives and mixtures thereof and the like.

Anti-acne actives can be effective in treating acne vulgaris, a chronic disorder of the pilosebaceous follicles. Non-limiting examples of useful anti-acne actives include the keratolytics such as salicylic acid (o-hydroxybenzoic acid), derivatives of salicylic acid such as 5-octanoyl salicylic acid and 4 methoxysalicylic acid, and resorcinol; retinoids such as retinoic acid and its derivatives (e.g., cis and trans); sulfur-containing D and L amino acids and their derivatives and salts, particularly their N-acetyl derivatives, mixtures thereof and the like.

Anti-microbial and anti-fungal actives can be effective to prevent the proliferation and growth of bacteria and fungi, especially in the after shave phase of the invention. Non-limiting examples of anti-microbial and anti-fungal actives include alum, boric acid, b-lactam drugs, quinolone drugs, ciprofloxacin, norfloxacin, tetracycline, erythromycin, amikacin, 2,4,4'-trichloro-2'-hydroxy diphenyl ether, 3,4,4'-Trichlorocarbanilide (triclocarban), phenoxyethanol, 2,4,4'-Trichloro-2'-Hydroxy Diphenyl Ether (triclosan); and mixtures thereof, and the like.

Anti-wrinkle, anti-skin atrophy and skin repair actives can be effective in replenishing or rejuvenating the epidermal layer. These actives generally provide these desirable skin care benefits by promoting or maintaining the natural process of desquamation. Non-limiting examples of anti-wrinkle and anti-skin atrophy actives include vitamins, minerals, and skin nutrients such as milk, vitamins A, E, and K; vitamin alkyl esters, including vitamin C alkyl esters; magnesium, calcium, copper, zinc and other metallic components; retinoic acid and its derivatives (e.g., cis and trans); retinal; retinol; retinyl esters such as retinyl acetate, retinyl palmitate, and retinyl propionate; vitamin B3 compounds (such as niacinamide and nicotinic acid), alpha hydroxy acids, beta hydroxy acids, e.g. salicylic acid and derivatives thereof (such as 5-octanoyl salicylic acid, heptyloxy 4 salicylic acid, and 4-methoxy salicylic acid); and mixtures thereof, and the like.

Skin barrier repair actives are those skin care actives which can help repair and replenish the natural moisture barrier function of the epidermis. Non-limiting examples of skin barrier repair actives include lipids such as cholesterol, ceramides, sucrose esters and pseudo-ceramides as described in European Patent Specification No. 556,957; ascorbic acid; biotin; biotin esters; phospholipids, and mixtures thereof, and the like.

Non-steroidal cosmetic soothing actives can be effective in preventing or treating inflammation of the skin. The soothing active enhances the skin appearance benefits of the present invention, e.g., such agents contribute to a more uniform and acceptable skin tone or color. Non-limiting examples of cosmetic soothing agents include the following categories: propionic acid derivatives; acetic acid derivatives; fenamic acid derivatives; mixtures thereof and the like. Many of these cosmetic soothing actives are described in U.S. Pat. No. 4,985,459 to Sunshine et al., issued Jan. 15, 1991, incorporated by reference herein in its entirety.

Artificial tanning actives can help in simulating a natural suntan by increasing melanin in the skin or by producing the appearance of increased melanin in the skin. Non-limiting examples of artificial tanning agents and accelerators include dihydroxyacetaone; tyrosine; tyrosine esters such as ethyl tyrosinate and glucose tyrosinate; and mixtures thereof, and the like.

Skin lightening actives can actually decrease the amount of melanin in the skin or provide such an effect by other mechanisms. Non-limiting examples of skin lightening actives useful.herein include aloe extract, alpha-glyceryl-L-ascorbic acid, aminotyrosine, ammonium lactate, glycolic acid, hydroquinone, 4 hydroxyanisole, and mixtures thereof, and the like.

Also useful herein are sunscreen actives. A wide variety of sunscreen agents are described in U.S. Pat. No. 5,087,445, to Haffey et al., issued Feb. 11, 1992; U.S. Pat. No. 5,073,372, to Turner et al., issued Dec. 17, 1991; U.S. Pat. No. 5,073,371, to Turner et al. issued Dec. 17, 1991; and Segarin, et al., at Chapter VIII, pages 189 et seq., of Cosmetics Science and Technology, all of which are incorporated herein by reference in their entirety. Non-limiting examples of sunscreens which are useful in the compositions of the present invention are those selected from octyl methoxyl cinnamate (Parsol MCX) and butyl methoxy benzoylmethane (Parsol 1789), 2-ethylhexyl p-methoxycinnamate, 2-ethylhexyl N,N-dimethyl-p-aminobenzoate, p- aminobenzoic acid, 2-phenylbenzimidazole-5-sulfonic acid, oxybenzone, and mixtures thereof, and the like.

Sebum stimulators can increase the production of sebum by the sebaceous glands. Non-limiting examples of sebum stimulating actives include bryonolic acid, dehydroetiandrosterone (DHEA), orizanol, and mixtures thereof, and the like.

Sebum inhibitors can decrease the production of sebum by the sebaceous glands. Non-limiting examples of useful sebum inhibiting actives include aluminum hydroxy chloride, corticosteroids, dehydroacetic acid and its salts, dichlorophenyl imidazoldioxolan (available from Elubiol), and mixtures thereof, and the like.

Optionally, other sebum inhibitory or antiperspirant astringent salts are included in the cleansing composition of the present invention. The astringent salts may be inorganic or organic salts of aluminum, zirconium, zinc and mixtures thereof. Preferably, the astringent salts are employed herein in particulate form, i.e., hydrophilic porous particles, of less than about 100 microns in size, preferably about 3 microns to about 10 microns in size. Salts useful as astringents or as components of astringent aluminum complexes include aluminum hydroxide, aluminum halides, aluminum hydroxyhalides, zirconyl oxyhalides, zirconyl hydroxyhalides and mixtures of these salt materials.

Aluminum salts of this type include aluminum chloride and the aluminum hydroxyhalides having the general formula $Al_2(OH)_xQ_y \cdot XH_2O$ where Q is chlorine, bromine or iodine, where x is 2 to 5 and x+y = 6 and x and y do not need to be integers; and where X is about 1 to 6. For example, aluminum chlorohydrate, having the formula $[Al_2(OH)_5Cl] \cdot XH_2O$, is preferred, due to its ready commercial availability and relatively low cost.

EP 1 868 778 B1

Several types of complexes utilizing the above astringent salts are known in the antiperspirant art. For example, U.S. Pat. No. 3,792,068 (Luedders et al.), discloses complexes of aluminum, zirconium and amino acids such as glycine. Complexes reported therein and similar structures are commonly known as ZAG. The ZAG complexes ordinarily have an Al:Zr ratio of from about 1.67 to 12.5 and a Metal:Cl ratio of from about 0.73 to 1. 93. The preferred amino acid for preparing such ZAG-type complexes is glycine of the formula $CH_2(NH_2)COOH$. Spherical ZAG, with particle size 1 to 100 microns, is especially preferred.

More specifically, the following is a list of astringent salts which may be useful for the present invention and which have approved listings under the United States Food & Drug Administration, Federal Register. They include aluminum chloride, aluminum chlorohydrate, aluminum chlorohydrex, aluminum chlorohydrex PEG, aluminum chlorohydrex PG, aluminum dichlorohydrate, aluminum dichlorohydrex PEG, aluminum dichlorohydrex PG, aluminum sesquichlorohydrate, aluminum sesquichlorohydrex PEG, aluminum sesquichlorohydrex PG, aluminum sulfate, aluminum zirconium octachlorohydrate, aluminum zirconium octachlorohydrex GLY (abbreviation for glycine), aluminum zirconium pentachlorohydrate, aluminum zirconium pentachlorohydrex GLY, aluminum zirconium tetrachlorohydrate, aluminum zirconium trichlorohydrate, aluminum zirconium tetrachlorohydrate GLY, and aluminum zirconium trichlorohydrate GLY.

Also suitable are: potassium aluminium sulphate, also known as alum $KAl(SO_4)_2 12H_2O$), aluminium undecylenoyl collagen amino acid, sodium aluminium lactate+aluminium sulphate $Al_2(SO_4)_3+Na_2HAl$ $(OOCCHOHCH_3)_2-(OH)_6$), sodium aluminium chlorohydroxylactate, aluminium bromohydrate ($Al_2Br(OH)_5 nH_2O$), aluminium chloride ($AlCl_36H_2O$), complexes of zinc salt and of sodium salt, complexes of lanthanum and cerium, and the aluminium salt of lipoamino acids (R-CO-NH-CHR'-CO-OAl-(OH)$_2$ with R=C6/C11 and R'=amino acid).

Preferably, the antiperspirant is an aluminium salt and, more preferably, it is chosen from potassium aluminium sulphate (alum) and aluminium chlorohydrate. Amounts of the active astringent salt may range from about 0.000001 % to about 20 %, preferably from about 0.10 % to about 18 %, more preferably about 1 % to about 15 %, and optimally about 2 % to about 3 % by weight of the cleansing composition. Aluminum chlorohydrate, referred to herein in shortened form as ACH, is the most preferred astringent salt for the purposes of the present invention, due to its wide commercial availability and relatively low cost.

Also useful as actives in the present invention are protease inhibitors. Protease inhibitors can be divided into two general classes: the proteinases and the peptidases. Proteinases act on specific interior peptide bonds of proteins, and peptidases act on'peptide bonds adjacent to a free amino or carboxyl group on the end of a protein and thus cleave the protein from the outside. The protease inhibitor suitable for use in the present invention include, but are not limited to, proteinases such as serine proteases, metalloproteases, cysteine proteases, and aspartyl protease, and peptidases, such as carboxypepidases, dipeptidases and aminopepidases, and mixtures thereof and the like.

Other useful active ingredients in the present invention are skin tightening agents. Non-limiting examples of skin tightening agents which are useful in the compositions of the present invention include monomers which can bind a polymer to the skin such as terpolymers of vinylpyrrolidone, (meth)acrylic acid and a hydrophobic monomer comprised of long chain alkyl (meth)acrylates, and mixtures thereof, and the like.

Active ingredients in the present invention may also include anti-itch ingredients. Suitable examples of anti-itch ingredients which are useful in the compositions of the present invention include hydrocortisone, methdilizine and trimeprazine, mixtures thereof, and the like.

Non-limiting examples of hair growth inhibitors which are useful in the compositions of the present invention include 17 beta estradiol, anti-angiogenic steroids, curcuma extract, cycloxygenase inhibitors, evening primrose oil, linoleic acid and the like. Suitable 5-alpha reductase inhibitors include ethynylestradiol, genistine and mixtures thereof, and the like.

Non-limiting examples of desquamating enzyme enhancers which are useful in the compositions of the present invention include alanine, aspartic acid, N methyl serine, serine, trimethyl glycine, mixtures thereof, and the like.

A non-limiting example of an anti-glycation agent which is useful in the compositions of the present invention would be Amadorine (available from Barnet Products Distributor), and the like.

## EXAMPLES

Except in the operating and comparative examples, or where otherwise explicitly indicated, all numbers in this description indicating amounts of material ought to be understood as modified by the word "about".

The following examples will more fully illustrate the embodiments of this invention. All parts, percentages and proportions referred to herein and in the appended claims are by weight unless otherwise illustrated. Physical test methods are described below.

Example 1

Useful examples of solid cleansing components for the razor head A - D according to the present invention may be prepared as shown in table 1 using the extrusion and stamping processing methods given below.

Table 1. Inventive solid cleansing compositions

| Ingredients | A | B | C | D |
|---|---|---|---|---|
| Sodium cocyl isethionate | 45 | 45 | 49.5 | 50 |
| Bentonite | 5 | | | |
| Starch | | 5 | | |
| Polymer JR (1) | | | 0.5 | |
| Sodium Isethionate | 10 | 10 | 10 | 10 |
| Stearic acid | 23 | 23 | 23 | 23 |
| Coco betaine | 3 | 3 | 3 | 3 |
| 82/18 tallow/coco Soap | 4 | 4 | 4 | 4 |
| Preservatives/Opacifiers | 0.5 | 0.5 | 0.5 | 0.5 |
| Sodium Stearate | 1.5 | 1.5 | 1.5 | 1.5 |
| Coconut fatty acid | 2 | 2 | 2 | 2 |
| Perfume | 1 | 1 | 1 | 1 |
| Water | 5 | 5 | 5 | 5 |
| Total | 100 | 100 | 100 | 100 |
| (1) Amerchol Corp. (Edison, NJ) | | | | |

Example 2

Useful examples of solid cleansing components for the razor head E - H according to the present invention may be prepared as shown in table 2 using the extrusion and stamping processing methods given below:

Table 2. Inventive solid cleansing compositions

| Ingredients | E | F | G | H |
|---|---|---|---|---|
| SCI | 35.5 | 32.5 | 29.5 | 37 |
| Alfa C14-16 Olefin Sulfonate | 3 | | 3 | 3 |
| Starch | | 5 | | |
| Kaolin | | | 5 | |
| Sodium Lauryl Sulfate | | | | 2.5 |
| Coco sulfosuccinate | | 5 | | |
| Stearic Acid | 25 | 20 | 25 | 20 |
| Sodium Stearate | 11 | 10 | 10 | 10 |
| Titanium Dioxide | 0.5 | 0.5 | 0.5 | 0.5 |
| 82/18 Coco/Tallow Soap | 13 | 15 | 15 | 15 |
| Sodium Isethionate | 5 | 5 | 5 | 5 |
| Water | 6 | 6 | 6 | 6 |
| Perfume | 1 | 1 | 1 | 1 |

Example 3

A useful solid cleansing component for the razor head I - L according to the present invention may be prepared as shown in table 3 using the melt cast processing methods given below:

Table 3 Inventive melt cast solid cleansing compositions

| Ingredients | I | J | K | L |
|---|---|---|---|---|
| Sodium Cocoyl Isethionate | 25 | 23.81 | 22.73 | 21.74 |
| Alpha C14-16 Olefin Sulfonate | 9 | 8.57 | 8.18 | 7.83 |
| SLES (2EO) | 9 | 8.57 | 8.18 | 7.83 |
| Propylene Glycol | 7 | 6.67 | 6.36 | 6.09 |
| Glycerin | 7 | 6.67 | 6.36 | 6.09 |
| 12- Hydroxystearic Acid | 14 | 13.33 | 12.73 | 12.17 |
| Lauryl Alcohol | | 4.76 | 9.09 | 13.04 |
| Sunflower Seed Oil | 25 | 23.81 | 22.73 | 21.74 |
| Water | 4 | 3.81 | 3.64 | 3.47 |
| Total | 100 | 100 | 100 | 100 |

Example 4

The mildness of solid cleansing composition M for the inventive razor head prepared from the formula shown in table 4 below was compared to comparative case 1A described in table la according to the Flex wash method described below (with eleven panellists). The inventive.composition was found to be substantially milder than the Comparative case where the average scores were found to be 0.23 for the Inventive case vs. 0.62 for the Comparative case. Composition M was prepared via the melt cast method described below.

Table 4. Inventive solid cleansing composition M

| Components | M |
|---|---|
| Sodium Cocoyl Isethionate | 21.74 |
| Stearic acid | 8.00 |
| Glycerin | 4.00 |
| Propylene Glycol | 4.00 |
| SLES-2EO 70 mole % | 4.00 |
| Sodium Hydroxide | 1.30 |
| 12- Hydroxystearic Acid | 9.00 |
| Alpha Olefin Sulfonate (C14-C18) | 3.00 |
| Cocoamidopropyl Betaine | 6.00 |
| Titanium Dioxide | 1.18 |
| Stearin (C16-C18 Triglyceride) | 4.00 |
| Sunflower Seed Oil | 16.00 |
| Petrolatum | 1.00 |
| Fragrance | 1.00 |
| Preservatives | 0.04 |
| Coconut Acid | 2.43 |

(continued)

| Components | M |
|---|---|
| Sodium Tallowate | 2.74 |
| Sodium Isethionate | 2.01 |
| Water | 5.13 |
| Sodium Stearate | 1.20 |
| Cocamidopropyl Betaine | 2.04 |
| Sodium Chloride | 0.19 |
| Total | 100.0 |

Example 5

Useful liquid cleansing compositions for the razor head O - Q according to the present invention, compared to a comparative liquid cleansing composition N, may be prepared as shown in table 5 using the liquid processing method given below:

Table 5

| | N | O | P | Q |
|---|---|---|---|---|
| **Ingredients** | Comp. | Inv. | Inv. | Inv. |
| Water | 55.2 | 50.4 | 50.3 | 50.2 |
| Sodium Laureth Sulfate | 15.0 | 15.0 | 10.0 | 5.0 |
| Potassium Cocoyl Isethionate | | 5.0 | 10.0 | 15.0 |
| Cocoamidopropyl betaine | 6.0 | 6.0 | 6.0 | 6.0 |
| Sodium chloride | 0.5 | 0.5 | 0.5 | 0.5 |
| Ethylene glycol monostearate | 1.5 | 1.5 | 1.5 | 1.5 |
| Perfume | 1.0 | 1.0 | 1.0 | 1.0 |
| Cationic guar polymer | 0.8 | 0.6 | 0.7 | 0.8 |
| Humectants (such as glycerin) | 20.0 | 20.0 | 20.0 | 20.0 |
| Total | 100.0 | 100.0 | 100.0 | 100.0 |

Processing method

Solid cleansing compositions A - L may be made by the following method.

The acyl isethionates may be made as follows. Sufficient amounts of coconut or other fatty acid and isethionate are combined in a vessel with a Lewis acid catalyst and heated to temperatures greater than 23°C to promote esterification. Once the required conversion is met, a vacuum is applied to the heated vessel so that any excess and unreacted fatty acid may be removed. The material may then be left to solidify or flashcooled into a mixer to begin the solid cleansing phase processing, described below.

Extruded bar process

Stearic acid is melted in a sigma or equivalent mixer. Approx. 25 % solution of sodium hydroxide is used for in-situ generation of sodium stearate. Enough time is provided to dissolve sodium stearate by heating the mass to about 120°C with continuous mixing. Once sodium stearate is dissolved, then the acyl isethionate such as sodium cocoyl isethionate is added and mixed. After that the rest of the ingredients are added and the moisture is reduced to about 5 % by wt. via conventional dehydration techniques. The blend is then cooled and solidified in a spray dryer or a chill roll. The chips are then mixed with fragrance and the skin conditioning and/or skin active agent and optionally triglyceride oil is blended in a chip mixer for about 5-10 minutes. This is followed by extrusion and stamping into the desired shape.

The stamped solid composition shape is inserted in the razor head.

Melt Cast process

The melt cast solid cleansing phase for the inventive razor head may be formulated depending on the melt properties of the particular blend used. In this'case all the bar ingredients, including exfoliants, are blended until uniform and finally at a temperature sufficient to render the blend flowable poured into a mold. The blend is then allowed to solidify under ambient or accelerated cooling conditions (such as refrigeration and the like).

Composition M is made by melt cast route. First 12-hydroxystearic acid is melted, then all the liquid components are added in the mixer except fragrance and sunflower seed oil. The mixer blend is heated to about 110˚C, and the acyl isethionate such as sodium cocoyl isethionate with the other additives are slowly dissolved. The homogeneous liquid is cooled to about 90˚C and optional skin conditioners such as sunflower seed oil are added. Perfume is added at about 75˚C and the melt is poured in the specified frame in the shaving device, and allowed to cool until hard.

Liquid cleansing phase process

The liquid or flowable cleansing phase for the inventive razor head may be formulated by blending the cleansing phase ingredients using any suitable blending technology until the composition is uniform, and the ingredients are dispersed or dissolved and adjusting the viscosity of the cleansing phase so that it is compatible with the liquid or flowable composition delivery system for the razor head. Appropriate mixing temperature can be 80˚C, with the addition of perfume at less than 40˚C. An example of a suitable mixer is IKA Labortechnik RW20. Advantageously the viscosity is kept below 2000 cps, more preferably below 1000 cps and most preferably below 600 cps measured at 25˚C using a Brookfield® or Haake® viscometer or any other suitable viscosity measurement method.

DESCRIPTION OF TEST METHODS

Methods of testing

One or more of the following tests can be used to characterize the cleansing phase of the inventive razor head and compare it to the cleansing phase of comparative razor heads.

a) Mildness test

i) FOREARM CONTROLLED APPLICATION TEST (FCAT) CLINICAL TEST METHODOLOGY

In this test the cleansing phase is tested outside of the razor head. This controlled washing test is similar to that described by Ertel et al (A forearm controlled application technique for estimating the relative mildness of personal cleansing products, J. Soc. Cosmet. Chem., 46, 67 (1995)).

Subjects report to the testing facility for the conditioning phase of the study, which consists of using an assigned marketed personal washing cleanser for general use at home, up to four days prior to start of the product application phase. On Day 1 of the product application phase, a visual assessment is made to determine subject qualification. Subjects must have dryness scores >1.0 and erythema scores >0.5, and be free of cuts and abrasions on or near the test sites to be included in the product application phase. Subjects who qualify to enter the product application phase will then be instructed to discontinue the use of the conditioning product and any other skin care products on their inner forearms, with the exception of the skin cleansing test formulations that are applied during the wash sessions.

Qualified subjects will then have four 3.0 cm diameter (round) evaluation sites marked on each of the forearms using a skin safe pen (a total of eight sites). Visual evaluations for erythema and dryness will be conducted immediately prior to the first wash in each session and again in the afternoon of the final day (Day 5).

Washing Procedure for Solid Cleansing Phase Products

1. Both arms are washed simultaneously. Test sites are treated in a sequential manner starting with the site closest to the flex area, ending with the site proximal to the wrist.

2. The sites closest to the flex area of the inner forearm of both the right and left arm are moistened with warm water (90˚-100˚F.).

3. A moistened Masslinn towel is rubbed in a circular motion on a wetted test bar for approximately 6 seconds by study personnel which will result in 0.2-0.5 g of product to be dispensed.

4. The site is washed with the designated product for 10 seconds followed by a 90 second lather retention phase.

5. The above procedure (1-4) is then repeated for each of the test sites. Sites are then be rinsed (e.g. using a temperature of 35˚C) for fifteen seconds and patted dry.

6. Upon completion the entire procedure is repeated (two washes/session).

For liquid cleansing phase products, a technician will prepare liquid products just prior to the wash session by dispensing between 0.1 g and 0.5 g of product either directly onto the skin or a moistened Maslinn towel, or an alternative application material. The washing procedure outlined above will then be used.

Evaluation Methods

Baseline visual assessments are made prior to the start of the product application phase, and immediately before each wash session thereafter, to evaluate dryness and erythema. The final visual evaluation is conducted on the afternoon of the final day.

The 0-6 grading scale shown in Table B is used to assess the test sites for dryness and erythema. To maintain the evaluator's blindness to product assignment, visual assessments are conducted in a separate area away from the product application area.

TABLE B Eythema and Dryness grading scale

| Grade | Erythema | Dryness |
|---|---|---|
| 0 | None | None |
| 1.0 | Barely perceptible | Patches of slight powderiness and redness occasional patches of small scales may be seen. Distribution generalized. |
| 2.0 | Slight redness | Generalized slight powderiness. Early cracking or occasional small lifting scales may be present. |
| 3.0 | Moderate redness | Generalized moderate powderiness and/or heavy cracking and lifting scales. |
| 4.0 | Heavy or substantial | Generalized heavy powderiness and/or redness heavy cracking and lifting scales. |
| 5.0 | Extreme redness | Generalized high cracking and lifting scales. Powderiness may be present but not prominent. May see bleeding cracks. |
| 6.0 | Severe redness | Generalized severe cracking. Bleeding cracks. Bleeding cracks may be present. Scales large, may be beginning to disappear. |

Instrumental readings are taken on the first (baseline) and final day of the study.
Mildness of test product is calculated as 1/(mean change in dryness at end of the study)
In addition to visual evaluation, instrumental assessments of the treated sites will be conducted using an evaporimeter and skin conductance meter as described in the reference above.

ii) Patch testing

48 hr continuous or 14 day cumulative insult patch test: In the 48 hr patch test 5 % to 15 % solution/slurry of the product is applied onto the upper arm/back of the subject using a standard cotton pad. Irritation response is recorded for up to 24 hrs after removal of the patch.. In the 14 day cumulative test a 5 % to 15 % solution/slurry of the product is applied repeatedly every 24 hrs for 14 days. Irritation response is recorded for up to 24 hrs after removal of patch.
Mildness of test product is evaluated as 1/(mean erythema at 24 hr after final patch removal).

b) Moisturization test

In this test the cleansing phase is tested outside of the razor head. Each outer, lower leg of a test subject will be divided into three sites, 6.35 x 6.35 cm (2.5 x 2.5 inch) squares (upper, middle and lower) for a total of 6 test sites per subject. One or two of the sites will be untreated, and will be included in the randomization of products. A technician will treat the sites once or twice with the designated amount of test material for 10 seconds. Cleansing products will remain on the test sites for a maximum of 90 seconds. Sites will be rinsed for 30 seconds each (e.g. using a temperature of 35˚C), ensuring that the test material from one site does not contaminate another site. After rinsing, the test sites are gently dried with a paper towel. The application consists of dosing with up to 5 different test cleansing phase materials on the designated sites, one material per test site, and one or two untreated sites. The study personnel will perform the following wash procedure.

Test Phase: Visual Evaluation

The scale as shown in Table C will be used to assess the test sites for dryness.

Table C

| Grade | Dryness Scale | Erythema Scale |
|---|---|---|
| 0.0 | No dryness | No erythema |
| 0.5 | Perceptible dryness, fine white lines | |
| 1.0 | Fine dry lines, white powdery look and/or some uplifting flakes, on less than 30 % of the test site | Mild erythema |
| 1.5 | More uniform flaking, covering 30 % to 50 % of the test site | |
| 2.0 | Uniform, marked flaking covering more than 50 % of the test site area and/or isolated scaling | Moderate confluent erythema |
| 2.5 | Slight to moderate scaling | |
| 3.0 | Moderate to severe scaling with some uplifting of the scales | Marked erythema |
| 3.5 | Severe scaling and/or slight fissuring | |
| 4.0 | Severe scaling and severe fissuring | Deep erythema |

Baseline visual assessments will be made prior to the start of the product application phase, and thereafter immediately before each of the instrumental assessments, to evaluate skin dryness and erythema. One trained evaluator will conduct all visual evaluations during the product application phase. The evaluator will examine both lower legs with the aid of an illuminated magnifying lamp with a 3 diopter lens and a shadow-free circular cool white fluorescent light source.

Instrumental Assessment

All instrumental evaluations will be taken following a 30 minute acclimation period. The indoor humidity and temperature data will be recorded and included in the final report. Instrumental measurements may be taken at some or all of the following time points: 0, 1, 2, 4, 6, 8 and 24 hours after product application. Instruments to be used with this protocol include: ServoMed Evaporimeter with EP1 or EP2 probe, Corneometer CM820, the Skicon Skin Hygrometer with the MT-8C probe, and the Moisture Checker. The room temperature will be maintained at 68˚ to 77˚F and 30 % to 40 % Relative Humidity.

Moisturization is defined as mean change from baseline of (visual dryness or skin hydration).

c) Moisturizer Deposition test

In this test the cleansing phase is tested outside of the razor head. Pre-condition the subject's skin (arms/legs) with non-moisturizer containing cleansing phase product for up to 2 days prior to testing. A baseline extraction is performed to estimate level of moisturizer (e.g. fatty acids) present on the skin prior to product application. Controlled single application of product to skin (arms or legs) is made. For wash, the cleansing phase is rubbed on skin for 30 secs and the lather left on for 90 secs, rinsed for 30 secs (e.g. using a temperature of 35˚C) then gently pat dry. Following this, the site is extracted using a suitable solvent (IPA)/methanol 1:1).

The extraction is performed as follows. A glass cup (3cm diameter) is placed on the skin. 3 mls of solvent is placed into this, and gently stirred with a glass rod for 2 minutes. The solvent is removed with a pipette. This step is repeated with a fresh 3 mls of solvent, to collect a total of 6 mls extract. The extracts are analyzed for stearic acid/palmitic acid content using either LC/MS or GC/MS, or the like.

### d) Skin abrasiveness test

In this test the cleansing phase is tested outside of the razor head. Skin abrasiveness is defined as consumer rated response of abrasivity on a 0-9 scale (0 means no abrasion, 10 is abrasivity caused by a pouf (i.e. a showering implement composed of thin plastic filaments, see also e.g. US Patent No. 5,650,384 to Gordon et al.).
This test is performed with 50 untrained consumers. They are asked to rate the abrasiveness of the test product on a 0-9 point scale. The data is normalized based on their response to a bar with no exfoliants which is assigned a value of zero and a pouf that is assigned a value of 9. The test products are applied to the flex area of the forearm by wetting the bar and rubbing back and forth 10-15 times.

### e) Cleansing efficacy test

In this test the cleansing phase is tested outside of the razor head. Model dirt (sebum/makeup - e.g. lipstick or mascara) is applied to a designated area on the forearm/face. The site is washed with the cleansing phase product. For wash, the bar is rubbed on skin for 1 minute, rinsed for 30 secs (e.g. using a temperature of 35°C), and gently pat dry. Amount of soil/makeup removed is estimated from the difference in the chromammeter readings using e.g. a Minolta Chromameter®, Model CM 2002 taken before and after wash. Alternately, high magnification digital mages are collected and analyzed using Optimas® software to quantitate the amount of soil/makeup removed during the wash.

### Make Up Application

Make up will be applied to the 3.5 x 2.5 cm marked area on the inner side of' the forearms in the manner consistent with its normal use. Cosmetic products are to be applied in a standardized way to ensure that approximately equal weights of make-up are transferred and that coverage of the test area is uniform. The application standards for the makeups are:

1.) Liquid make-up - 20 µl pipette to the site and spread uniformly with gloved index finger.
2.) Lipstick - Three overlapping swipes.
3.) Eye Color Stick - Three overlapping swipes.
4.) Mascara - spread uniformly using spatula for even coverage.

### Soil Application

Soils will be applied to the 3.5 x 2.5 cm marked area on the inner side of the forearms in the manner described below and is specific to each individual study if soils are being used. The application techniques for the soils are:

1.) Grease - 0.25g - 1.5g. will be applied.
2.) Food - 0.25g - 1.5g. will be applied.
3.) Protein - 0.25g - 1.5g. will be applied.

### Product Testing

Baseline measurements will be performed using the Minolta Chromameter CM-2002. Make-up or soil will then be applied to the delineated test sites as described above. Chromameter measurements will be taken again after the make up has dried for 10 minutes, then the make-up/soil will be removed. The standard washing procedure used to remove the make-up/soil is a 30 second wash with 0.5 cc of a liquid product with a 15 second rinse under running water using a suitable constant temperature (e.g. 35°C). When a towelette product is being used, the towelette is rubbed over the test site in a circular motion for 15 seconds. Final Chromameter measurement will be taken after the make-up/soil has been removed. This procedure may be performed twice a day for a period of up to 3 days. In repeat application, studies visual assessments will be made for dryness and erythema using the standard visual grading scale as described above.

### f) Skin smoothness

In this test the cleansing phase is tested outside of the razor head. Skin smoothness is evaluated (clinically) via Primos® (in-vivo optical skin topography measuring device supplied by GFM Esstezhnik GmbH, Berlin, Germany).

Baseline roughness is measured (on leg/arms - starting dryness around grade 1-2). For wash, bar rubbed on skin for 30 secs and the lather left on for 90 secs, rinsed for 30 secs at 35°C. Measure again the roughness 30 minutes after wash process. This procedure may be performed twice a day for a period of up to 5 days.

Smoothness is defined as the mean decrease in roughness at end of study period. Alternately skin smoothness can also be evaluated in a consumer test as follows.

The consumer test protocol consists of:

1) Recruiting aprox. 10-20 women in the age group of 25-65 and who are complexion bar users.
2) Use test and comparative products for a week each. Half the panellists would use the test product first, and the other half would use the comparative product first.
3) At the end of the test, the panellists rate their preference (on a 0-5 point scale) on the attribute of "Skin feels smoother".

Smoothness is defined as the consumer rating on the 0-5 point scale.

g) Skin softness

In this test the cleansing phase is tested outside of the razor head. Skin softness may be evaluated using the Linear Skin Rheometer (Goodyear Scientific Instruments, UK). Exfoliated skin has less dry flakes - hence is more soft/ less stiff. The test involves baseline skin rheometer readings (on the leg/arms) to measure the dynamic spring constant (mgf/mm) of skin which is related to skin stiffness/softness. For wash, the bar is rubbed on the skin for 30 seconds and the lather left on for 90 seconds, rinsed for 30 seconds (at a suitable temperature e.g. 35°C), and the skin is gently pat dry. Next measure skin stiffness/softness 30 minutes after wash. This procedure may be performed twice a day for a period of up to 5 days. Softness is defined as the mean decrease in dynamic spring constant during the study period observed during the study period.

Alternately skin softness can also be evaluated in a consumer test as follows:

The test protocol consists of:

1) Recruiting approx. 10-20 women in the age group of 25-65 and who are complexion bar users.
2) Use test and comparative products for a week each. Half the panellists would use the test product first and the other half would use the comparative product first.
3) At the end of the test, the panellists rate their preference (on a 0-5 point scale) on the attribute of "Skin feels softer".

Softness is defined as the consumer rating on the 0-5 point scale

h) pH test method

Form an aqueous slurry by blending 10 grams of the cleansing phase formula with 90 g of water to create a 10 % slurry. The pH of the slurry is then measured at 25°C.

i) Zein test method

The cleansing base of the inventive razor head preferably have zein solubility's of under about 50, 40, 30, and most preferably under about 25 using the zein solubility method set forth below. The lower the zein score, the milder the product is considered to be. This method involves measuring the solubility of zein (corn protein) in cleansing base solutions as follows:

0.3 g of cleansing base and 29.7 g of water are mixed thoroughly. To this is added 1.5 g of zein, and mixed for 1 hour. The mixture is then centrifuged for 30 minutes at 3000 rpm. After centrifugation, the pellet is extracted, washed with water, and dried in a vacuum oven for 24 hours until substantially all the water has evaporated. The weight of the dried pellet is measured and percent zein solubilized is calculated using the following equation:

$$\text{\% Zein solubilized} = 100 \ (1\text{-weight of dried pellet}/1.5).$$

The % Zein is further described in the following references: E. Gotte, Skin compatibility of tensides measured by their capacity for dissolving zein protein, Proc. IV International Congress of Surface Active Substances, Brussels, 1964, pp 83-90.

j) Solid cleansing phase mass sensory exfoliation index

For this test, the cleansing phase is tested outside of the razor head. The cleansing phase sensory exfoliation index is determined using the following procedure.

The user takes the solid cleansing phase mass in one hand and rotates it under running water at 35˚C. The number of rotations required for the exfoliant to be perceived (i.e. by tactile sensation) by the user is recorded. The solid cleansing phase mass exfoliation index is defined as the mean number of rotations required to perceive the exfoliant particles in the solid cleansing phase mass.

k) General Consumer Test Protocol

For this test, the cleansing phase is tested outside of the razor head. The test protocol consists of:

1) Recruiting approx. 10-20 women in the age group of 25-65 and who are complexion bar users.
2) Use test and comparative cleansing phase products for a week each. Half the panellists would use the test product first and the other half would use the comparative product first.
3) At the end of the test, the panellists rate their preference on a 0-5 point scale for the following attributes:

Exfoliates

Provides Gentle Exfoliation
Mositurizes and exfoliates
Skin feels softer
Skin feels smoother
Is good for dry skin

l) Flex wash test method

The samples are tested outside the razor heads. The study consists of three supervised daily washes in the morning (one hour apart) for three consecutive days of panel test subjects. Lather is applied to pre-moistened sponges by stroking them across wet sample bars ten times. Panellists wash their inner flex areas (inner arm between the wrist and elbow) in an elliptical motion for one minute, rinse, and pat dry. The test areas are evaluated for erythema at baseline, prior to each wash, and three hours after the third wash on each day. Visual grading is conducted using a five-point scale for erythema. The scale in table C is used with values that range from 0 (none) to 4 (severe) with 2 being the test endpoint score.at which point the test for that particular panellist is discontinued. The visual grading data is analyzed using the Wilcoxon Signed Rank procedure.

m) Mush test

This test is used for the solid cleansing phase tested outside of the razor head. Shave the solid cleansing phase mass to the dimensions of 7cm x 4cm x 2cm and carve a line halfway down the center of the solid cleansing phase mass (at the 3.5 cm mark). Measure the weight of the solid cleansing phase mass. Suspend half of the solid cleansing phase mass (3.5 cm) in deionized water for 2 hours at a temperature of 25˚C. After this time, lift up the solid cleansing phase mass and remove excess water by suspending the solid cleansing phase mass for 30 seconds, then weigh the solid cleansing phase mass. This is the weight of the solid cleansing phase mass, the mush, and the absorbed water.

After weighing, lightly scrape off the mush from the solid cleansing phase mass, being careful not to scrape off excess solid cleansing phase mass material that is not mush. Discard the mush and let the solid cleansing phase mass dry for 12 hours. Weigh the final dry solid cleansing phase mass and the difference of the initial dry solid cleansing phase mass and the final dry solid cleansing phase mass, calculated for the 50 cm$^2$ solid cleansing phase mass surface area, is the amount of mush (grams). The difference in weight of the soaked solid cleansing phase mass and the initial dry solid cleansing phase mass is the amount of water absorbed.

The Mush Factor is defined as the ratio of the mush/50 cm$^2$ of a given solid cleansing phase mass to a control mild isethionate solid cleansing phase mass mush/50 cm$^2$ or in the present case, formula E provided above. For example, the mush of the inventive solid cleansing phase mass (formula A) is 6.2g mush/50 cm$^2$, and the mush of formula E is 10.1 g mush/50 cm$^2$ to provide a Mush Factor of 0.61.

n) <u>Wear rate method</u>

This test is used for the solid cleansing phase tested outside of the razor head.

Weigh the solid cleansing phase mass to be tested. Set up an 8 liter bucket with continuous water running through it at 40.5˚C. Immerse the solid cleansing phase mass and rotate it in the hands 20 times. Repeat. Immerse the solid cleansing phase mass again to remove adhering lather, and let dry in the air at 25˚C and approximately 50 % RH in a dish. Repeat every two hours over an 8 hour span. Let dry for 12 hours at 25˚C and approximately 50 % RH in a dish, and repeat for another 8 hour span.

Add 10 g of deionized water to the dish between immersions, and while the solid cleansing phase mass is resting in the dish, (this should be additive over the 8-hour span - meaning that after the first 2 hours 10g water are added to the dish; after 4 hours, add 10 grams more, totaling 20 grams water in the dish. After 6 hours, add 10 grams more, totaling 30g water in the dish, and so on for the 8-hour period, then let dry for 12 hours. The weight of the solid cleansing phase mass after 12 hours is recorded and the wear rate is the percent weight loss of the solid cleansing phase mass.

o) <u>Krafft point determination</u>

Make up a 10 % by wt. solution of surfactant or other sample in water. If needed, heat the system to dissolve the sample completely. Transfer the clear solution to a glass test tube. Place the test tube in a beaker equipped with a stirrer and filled with sufficient water to evenly cool the surfactant or sample solution. The solution should be cooled with continuous stirring and the temperature should be continuously recorded. Note the temperature when the crystallization process begins such that the solution becomes turbid. This temperature is taken as the Krafft point. If the crystallization temperature is below room temperature, add ice to the beaker to cool the test tube below room temperature to measure the subambient Krafft point.

p) <u>Method for calculation of yield stress with cheese cutter device</u>

This test is used for the solid cleansing phase tested outside of the razor head. An approximate value for yield stress can be determined by the cheese cutter method. The principle of the measurement is that a wire penetrating into a material with a constant force will come to rest when the force on the wire due to stress balances the weight. The force balance is:

$$\text{Weight driving wire = force on wire due to material stress}$$

$$m\ g = K\ ys\ l \cdot D$$

where

m = mass driving wire (actual mass used in calculation is the mass placed on the device plus the weight of the arm which adds to the extra weight on the sample)
g = gravitational constant, 9.8 m/sec$^2$
ys = yield stress
l = length of penetration of wire into soap after 1 minute (mm)
D = diameter of wire (mm)
K = a geometrical constant

The final equation is:

$$ys = (3/8)\ m\ g\ /(l\ D)$$

<u>Procedure</u>

Cut a square of solid cleansing phase and position on the yield stress device. Place a mass on the yield stress device while holding the arm. 400g is an appropriate mass, although less might be needed for a very soft material. Gently lower the arm so the wire just touches the soap and let the arm go. Stop the vertical motion of the arm after one minute, and push the soap through the wire horizontally to cut a wedge out of the sample. Take the mass off the device and then measure the length of the cut in the sample. The wire would continue to cut the soap at a slow rate, but the length of the cut made by the wire in one minute is taken as the final value.. Measure the temperature of the soap while the test proceeds.

Sample calculation:

A 400 gram weight is used on the yield stress device and a 22 mm slice is measured where the wire has cut the solid cleansing phase after 1 minute. Assuming the diameter of the wire is 0.6 mm, the approximate yield stress is:

$$\frac{(3/8)(400+56)[g]9.8[m/sec^2]10^{-3}[kg/g]}{22[mm]0.6[mm]10^{-6}[m^2/mm^2]} = 1.3105Pa \text{ or } 130kPa$$

Optionally an Instron testing device (supplied by Instron Co., Boston, MA) may be used instead of a weight to apply stress to the wire contacting the solid cleansing phase mass.

q) Viscosity Measurement

Scope

This method is suitable for the measurement of the viscosity of the liquid or flowable cleansing composition.

Apparatus

Brookfield Cone and Plate DV-II+ Viscometer:;
Spindle S41;

Procedure

1. Turn on Water Bath attached to the sample cup of the viscometer. Make sure that it is set for 25˚C. Allow temperature readout to stabilize at 25˚C before proceeding.

2. With the power to the viscometer off, remove the spindle (S41) by turning counterclockwise.

3. Turn the power on and press any key as requested to autozero the viscometer.

4. When the autozero function is complete, replace the spindle (turning clockwise) and press any key.

5. Attach the sample cup. Using the up/down arrow keys, slowly change the speed to 10 rpm and press the SET SPEED key. Use the SELECT DISPLAY key so that the display is in % mode.

6. Turn the motor on. If the display jumps to 0.4 % or higher or will not settle to $0 \pm 0.1$ %, turn the adjustment ring clockwise until it does.

7. Rotate the adjustment ring counterclockwise until the reading is fluctuating between 0.0 and 1.0 %. The fluctuation must occur approximately every 6 seconds.

8. Turn the adjustment ring clockwise exactly the width of one division from the setting reached in step 7.

9. Turn the motor off. Using the up/down arrow keys, slowly change the speed to 0.5 rpm and press the SET SPEED key. Use the SELECT DISPLAY so that the display is in cP.

10. Place $2 \pm 0.1$g of product to be measured into the sample cup. Attach the cup to the viscometer.

11. Allow the product to remain in the cup with the motor OFF for 2 minutes.

12. Turn the motor ON and allow the spindle to turn for 2 minutes before noting the reading on the display.

While this invention has been described with respect to particular embodiments thereof, it is apparent that numerous other forms and modifications of the invention will be obvious to those skilled in the art. The appended claims and this invention generally should be construed to cover all such obvious forms and modifications which are within the true spirit and scope of the present invention.

**Claims**

1. A razor head for personal shaving, comprising:

 a) at least one blade member;
 b) a first skin engaging portion effectively positioned adjacent to the at least one blade for treating the skin of a user in advance of or simultaneously with the blade member contacting the skin of a user, the portion including and/or fluidly communicating with a mild cleansing base; and
 c) wherein the cleansing base is a solid and includes 1 - 60% wt surfactant selected from C8 - C18 acyl isethionate (s); wherein the total C8 - C18 acyl isethionates comprises 5 to 40% wt C16 - C18 acyl isethionates and from 40 - 95% wt C8 - C14 acyl isethionates based on the total acyl isethionates.

2. The razor head of claim 1, further comprising a second skin engaging portion effectively positioned adjacent to the at least one blade member for treating the skin of a user subsequent to and/or simultaneously with the blade member contacting the skin of a user, the portion including and/or fluidly communicating with an after shave base.

3. The razor head of claim 1 or claim 2, wherein the at least one blade member and the cleansing base are situated within a single frame or in a plurality of frames positioned substantially adjacent to each other.

4. The razor head of any one of the preceding claims, wherein the cleansing phase has a zein value of less than 50.

5. The razor head of any one of the preceding claims, wherein the cleansing phase includes one or more hydrophillic and/or hydrophobic skin conditioning compounds in a total concentration range of 1 to 60 % by wt.

6. The razor head of any one of the preceding claims, wherein the cleansing phase is in the form of a solid having a yield stress value from 20 Kpa to 400 KPa at 25˚C and 50 % RH.

7. The razor head of claim 6, wherein the isethionate surfactant has a Krafft point of 20˚C or greater.

8. The razor head of any one of the preceding claims, wherein the cleansing phase contains an effective concentration of at least one compound selected from anti-acne actives, anti-wrinkle and anti-skin atrophy actives, skin barrier repair aids, cosmetic soothing aids, topical anesthetics, artificial tanning agents and accelerators, skin lightening actives, antimicrobial and antifungal actives, sunscreen actives, sebum stimulators, sebum inhibitors, antiperspirants and anti-glycation actives, or mixtures thereof.

9. The razor head of any one of claims 2 to 8, wherein the after shave phase contains an effective concentration of at least one compound selected from anti-acne actives, anti-wrinkle and anti-skin atrophy actives, skin barrier repair aids, cosmetic soothing aids, topical anesthetics, artificial tanning agents and accelerators, skin lightening actives, antimicrobial and antifungal actives, sunscreen actives, sebum stimulators, sebum inhibitors, antiperspirants or anti-glycation actives, or mixtures thereof.

10. The razor head of any one of claims 5 to 9, wherein the skin moisturising compound is selected from fatty acids, glyceride oil, mineral oil, petrolatum, glycerin, polyethylene glycol, or blends thereof.

11. The razor head of any one of the preceding claims, wherein the cleansing base provides an aqueous slurry pH of 4 to 8.

12. The razor head of any one of the preceding claims, further comprising less than 10 % by wt. of soluble soap.

**Patentansprüche**

1. Rasierkopf zur Körperrasur, umfassend:

   a) wenigstens ein Klingenelement;
   b) einen ersten die Haut berührenden Teil, der effektiv angrenzend an die wenigstens eine Klinge positioniert ist, zur Behandlung der Haut eines Benutzers vor oder gleichzeitig mit dem In-Kontakt-Kommen des Klingenelements mit der Haut eines Benutzers, wobei der Teil eine milde Reinigungsgrundlage enthält und/oder mit einer mildern Reinigungsgrundlage in Fließverbindung steht, und
   c) wobei die Reinigungsgrundlage ein Feststoff ist und 1 - 60 Gewichtes-% Tensid, ausgewählt aus C8-C18-Acylisethionat(en), umfasst, wobei die gesamten C8-C18-Acylisethionate 5 bis 40 Gewichts-% C16-C18-Acylisethionate und 40 - 95 Gewichts-% C8-C14-Acylisethionate, bezogen auf die gesamten Acylisethionate, umfassen.

2. Rasierkopf gemäß Anspruch 1, außerdem umfassend einen zweiten die Haut berührenden Teil, der effektiv angrenzend an die wenigstens eine Klinge positioniert ist, zur Behandlung der Haut eines Benutzers nach und/oder gleichzeitig mit In-Kontakt-Kommen des Klingenelements mit der Haut eines Benutzers, wobei der Teil eine After-Shave-Grundlage enthält und/oder mit einer After-Shave-Grundlage in Fließverbindung steht.

3. Rasierkopf gemäß Anspruch 1 oder Anspruch 2, wobei das wenigstens eine Klingenelement und die Reinigungsgrundlage sich in einem einzelnen Rahmen oder in einer Vielzahl von Rahmen, die im Wesentlichen aneinander angrenzend positioniert sind, befinden.

4. Rasierkopf gemäß einem der vorangehenden Ansprüche, wobei die Reinigungsphase einen Zein-Wert von kleiner als 50 hat.

5. Rasierkopf gemäß einem der vorangehenden Ansprüche, wobei die Reinigungsphase eine oder mehrere hydrophile und/oder hydrophobe Hautkonditionierende Verbindungen in einem Gesamtkonzentrationsbereich von 1 bis 60 Gewichts-% umfasst.

6. Rasierkopf gemäß einem der vorangehenden Ansprüche, wobei die Reinigungsphase in der Form eines Feststoffs ist, der bei 25 °C und 50 % relativer Feuchtigkeit einen Fließgrenzwert von 20 Kpa bis 400 KPa hat.

7. Rasierkopf gemäß Anspruch 6, wobei das Isethionat-Tensid einen Krafft-Punkt von 20 °C oder höher hat.

8. Rasierkopf gemäß einem der vorangehenden Ansprüche, wobei die Reinigungsphase eine wirksame Konzentration wenigstens einer Verbindung enthält, die ausgewählt ist aus Wirkstoffen gegen Akne, Wirkstoffen gegen Falten und gegen Hautatrophie, Hautbarriere-Reparaturhilfsmitteln, kosmetischen Glättungshilfsmitteln, topischen Anästhetika, künstlichen Bräanungsmitteln und - beschleunigern, Wirkstoffen zur Hautaufhellung, antimikrobiellen und antifungalen Wirkstoffen, Sonnenschutzwirkstoffen, Sebum-Stimulatoren, Sebum-Inhibitoren, Antitranspirantien und Antiglykationswirkstoffen oder Gemischen davon.

9. Rasierkopf gemäß einem der Ansprüche 2 bis 8, wobei die After-Shave-Phase eine wirksame Konzentration wenigstens einer Verbindung enthält, die ausgewählt ist aus Wirkstoffen gegen Akne, Wirkstoffen gegen Falten und gegen Hautatrophie, Hautbarriere-Reparaturhilfsmitteln, kosmetischen Glättungshiffismitteln, topischen Anästhetika, künstlichen Bräunungsmitteh und -beschleunigern, Wirkstoffen zur Hautaufihellung, antimikrobiellen und antifungalen Wirkstoffen, Sannenschutzwirkstoffien, Sebum-Stimulatoren, Sebum-Inhibitoren, Antitranspirantien und Antiglykationswirkstoffen oder Gemischen davon.

10. Rasierkopf gemäß einem der Ansprüche 5 bis 9, wobei die Haut-feuchtmachende Verbindung ausgewählt ist aus Fettsäuren, Glyceridöl, Mineralöl, Petrolatum, Glycerin, Polyethylenglykol und Mischungen davon.

11. Rasierkopf gemäß einem der vorangehenden Ansprüche, wobei die Fteinigungsgrundiage einen pH einer wässrigen Aufschlämmung von 4 bis 8 bereitstellt.

12. Rasierkopf gemäß einem der vorangehenden Ansprüche, der außerdem weniger als 10 Gewichts-% lösliche Seife umfasst.

**Revendications**

1.  Tête de rasoir pour rasage personnel, comprenant :

    a) au moins un élément de lame ;
    b) une première partie de contact avec la peau positionnée efficacement de façon adjacente à l'au moins une lame pour traiter la peau d'un utilisateur avant ou en même temps que l'élément de lame entre en contact avec la peau d'un utilisateur, la partie comprenant et/ou communiquant de façon fluide avec une base de nettoyage douce ; et
    c) dans laquelle la base de nettoyage est un solide et comprend 1 à 60 % en poids de tensio-actif sélectionné parmi un ou des iséthionate(s) d'acyle C8 - C18 ; dans laquelle les iséthionates d'acyle C8 - C18 totaux comprennent de 5 à 40 % en poids d'iséthionates d'acyle C16 - C18 et de 40 à 95 % en poids d'iséthionates d'acyle C8 - C14 sur la base des iséthionates d'acyle totaux.

2.  Tête de rasoir selon la revendication 1, comprenant en outre une seconde partie de contact avec la peau efficacement positionnée de façon adjacente à l'au moins un élément de lame pour traiter la peau d'un utilisateur après et/ou en même temps que l'élément de lame entre en contact avec la peau d'un utilisateur, la partie comprenant et/ou communiquant de façon fluide avec une base d'après-rasage.

3.  Tête de rasoir selon la revendication 1 ou la revendication 2, dans laquelle l'au moins un élément de lame et la base de nettoyage sont situés à l'intérieur d'un cadre unique ou dans une pluralité de cadres positionnés de façon sensiblement adjacente les uns par rapport aux autres.

4.  Tête de rasoir selon une quelconque des revendications précédentes, dans laquelle la phase de nettoyage possède une valeur de zéine inférieure à 50.

5.  Tête de rasoir selon une quelconque des revendications précédentes, dans laquelle la phase de nettoyage comprend un ou plusieurs composés hydrophiles et/ou hydrophobes de conditionnement de peau dans une plage de concentration totale de 1 à 60 % en poids.

6.  Tête de rasoir selon une quelconque des revendications précédentes, dans laquelle la phase de nettoyage est sous forme de solide possédant une valeur de limite d'élasticité de 20 Kpa à 400 KPa à 25˚C et 50 % HR.

7.  Tête de rasoir selon la revendication 6, dans laquelle le tensio-actif d'iséthionate possède un point de Krafft de 20˚C ou plus.

8.  Tête de rasoir selon une quelconque des revendications précédentes, dans laquelle la phase de nettoyage contient une concentration efficace d'au moins un composé sélectionné parmi des agents actifs anti-acné, des agents actifs antirides et anti-atrophie cutanée, des aides de réparation de barrière cutanée, des aides calmantes cosmétiques, des anesthésiques topiques, des agents et accélérateurs de bronzage artificiel, des agents actifs éclaircissants de peau, des agents actifs antimicrobiens et antifongiques, des agents actifs écrans solaires, des stimulateurs de sébum, des inhibiteurs de sébum, des agents antiperspirants et des agents actifs anti-glycation, ou des mélanges de ceux-ci.

9.  Tête de rasoir selon une quelconque des revendications 2 à 8, dans laquelle la phase d'après-rasage contient une concentration efficace d'au moins un composé sélectionné parmi des agents actifs anti-acné, des agents actifs antirides et anti-atrophie cutanée, des aides de réparation de barrière cutanée, des aides calmantes cosmétiques, des anesthésiques topiques, des agents et accélérateurs de bronzage artificiel, des agents actifs éclaircissants de peau, des agents actifs antimicrobiens et antifongiques, des agents actifs écrans solaires, des stimulateurs de sébum, des inhibiteurs de sébum, des agents antiperspirants et des agents actifs anti-glycation, ou des mélanges de ceux-ci.

10. Tête de rasoir selon une quelconque des reven-dications 5 à 9, dans laquelle le composé hydratant pour la peau est sélectionné parmi des acides gras, de l'huile de glycéride, de l'huile minérale, du gel de paraffine, de la glycérine, du polyéthylène-glycol, ou des mélanges de ceux-ci.

11. Tête de rasoir selon une quelconque des revendications précédentes, dans laquelle la base de nettoyage fournit un pH de pâte aqueuse de 4 à 8.

12. Tête de rasoir selon une quelconque des revendications précédentes, comprenant en outre moins de 20 % en poids de savon soluble.

Fig.1.

Fig.2.

Fig.3.

Fig.4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4944090 A, Sumnall
- US 4074429 A, Roberts
- US 6584690 B, Orloff
- US 20050011073 A, Sandor
- US 4170821 A, Booth
- US 20040181943 A, Kwiecken
- US 5389279 A, Au
- US 5009814 A, Kelkenberg
- US 3723325 A, Parran Jr.
- EP 556957 A
- US 4985459 A, Sunshine
- US 5087445 A, Haffey
- US 5073372 A, Turner
- US 5073371 A, Turner
- US 3792068 A, Luedders
- US 5650384 A, Gordon

### Non-patent literature cited in the description

- **Segarin et al.** Cosmetics Science and Technology. 189
- **Ertel et al.** A forearm controlled application technique for estimating the relative mildness of personal cleansing products. *J. Soc. Cosmet. Chem.,* 1995, vol. 46, 67
- **E. Gotte.** Skin compatibility of tensides measured by their capacity for dissolving zein protein. *Proc. IV International Congress of Surface Active Substances,* 1964, 83-90